# EUROPÄISCHE PATENTSCHRIFT

(11) **EP 3 737 943 B1**
(45) Veröffentlichungstag und Bekanntmachung des Hinweises auf die Patenterteilung: **04.09.2024**
(21) Anmeldenummer: 19700016.9
(22) Anmeldetag: 02.01.2019
(51) Int. Cl.: G01N 33/36, B65H 59/40

(54) **VERFAHREN UND VORRICHTUNG ZUR ÜBERWACHUNG EINES TEXTURIERPROZESSES**
METHOD AND DEVICE FOR MONITORING A TEXTURING PROCESS
PROCÉDÉ ET DISPOSITIF POUR SURVEILLER UN PROCESSUS DE TEXTURATION

(30) Priorität: 09.01.2018 DE 102018000124
(43) Veröffentlichungstag der Anmeldung: 18.11.2020
(73) Patentinhaber: Oerlikon Textile GmbH & Co. KG, 42897 Remscheid (DE)
(72) Erfinder: HUTHMACHER, Jörg, 45768 Marl (DE)
(74) Vertreter: Neumann, Ditmar
(86) Internationale Anmeldenummer: PCT/EP2019/050015
(87) Internationale Veröffentlichungsnummer: WO 2019/137835

(56) Entgegenhaltungen:
- WO-A1-2015/052624
- WO-A1-2018/224398
- DE-A1- 10 237 978
- US-A- 5 682 146

## Beschreibung

Die Erfindung betrifft ein Verfahren zur Überwachung eines Texturierprozesses zur Herstellung gekräuselter Fäden gemäß dem Oberbegriff des Anspruchs 1 sowie eine Vorrichtung zur Überwachung des Texturierprozesses gemäß dem Oberbegriff des Anspruchs 11.

Ein gattungsgemäßes Verfahren und eine gattungsgemäße Vorrichtung zur Überwachung eines Texturierprozesses zur Herstellung von gekräuselten Fäden sind beispielsweise aus der DE 196 14 027 A1 bekannt.

Beim Texturieren synthetischer Fäden ist es üblich, den Herstellungsprozess kontinuierlich zu überwachen, um so eine mögliche stabile Prozessführung und insbesondere möglichst eine stabile Produktqualität an dem gekräuselten Faden zu erhalten. Hierbei hat sich die Überwachung einer Fadenspannung an dem laufenden Faden bewährt, um Prozessstörungen und/oder Produktschwankungen zu erkennen. Bei dem bekannten Verfahren und der bekannten Vorrichtung zur Überwachung des Texturierprozesses wird hierzu die Fadenspannung an dem Faden fortlaufend gemessen. Die dabei erzeugten Messsignale der Fadenspannung werden mit einem Schwellwert einer zulässigen Fadenspannung verglichen. Hierbei zeigt der Signalverlauf der in einem Zeitintervall kontinuierlich erfassten Messsignale der Fadenspannung mögliche Toleranzabweichungen an. In Abhängigkeit von der jeweiligen Störung im Prozess können hierbei unterschiedliche Signalverläufe der Messsignale erfasst werden. So können erfahrene Operatoren einen Messsignalverlauf der Fadenspannung dazu nutzen, um mögliche Störquellen des Texturierprozesses zu identifizieren.

Das bekannte Verfahren und die bekannte Vorrichtung zur Überwachung des Texturierprozesses besitzen jedoch den großen Nachteil, dass nur die Messsignalverläufe zur Analyse herangezogen werden, die einen Schwellwert der Fadenspannung überschreiten. Damit sind bereits unzulässige Produktgrenzen erreicht, die zu einer mangelhaften Qualität des Fadens führen. Darüberhinaus besitzt das bekannte Verfahren und die bekannte Vorrichtung den weiteren Nachteil, dass eine Identifizierung von möglichen Störquellen allein von der Erfahrung des jeweiligen Operators abhängt.

Auch aus der US 5,682,146 A und der nachveröffentlichten WO 2018/224398 A1 sind Verfahren zur Prozessüberwachung bekannt.

Es ist daher Aufgabe der Erfindung, das gattungsgemäße Verfahren und die gattungsgemäße Vorrichtung zur Überwachung eines Texturierprozesses zur Herstellung von gekräuselten Fäden derart weiterzubilden, dass eine verbesserte Prozessführung zur Herstellung gleichmäßiger Fadenqualitäten möglich wird.

Ein weiteres Ziel der Erfindung liegt darin, ein gattungsgemäßes Verfahren und eine gattungsgemäße Vorrichtung zur Überwachung eines Texturierprozesses zu schaffen, mit welchem es möglich ist, Prozessstörungen möglichst frühzeitig zu identifizieren sowie schnell und gezielt zu beseitigen.

Diese Aufgabe wird erfindungsgemäß durch ein Verfahren mit den Merkmalen nach Anspruch 1 und durch eine Vorrichtung mit den Merkmalen nach Anspruch 11 gelöst.

Vorteilhafte Weiterbildungen der Erfindung sind durch die Merkmale und Merkmalskombinationen der jeweiligen Unteransprüche definiert.

Die Erfindung basiert auf der Erkenntnis, dass die in einem Zeitintervall auftretende Folge von Messsignalen bestimmte Charakteristiken aufweist, die sich in den Signalverläufen darstellen und jeweils als Indiz einer Prozesssteuerung genutzt werden können. Somit wird erfindungsgemäß die im Zeitintervall auftretende Folge der Messsignale der Fadenspannung zur frühzeitigen Diagnose einer von mehreren Störquellen mit einem maschinellen Lernprogramm analysiert. So lassen sich bereits frühzeitig bestimmte in einer Zeitfolge auftretende Messwertveränderungen der Fadenspannung aufgrund einer bekannten Störquelle frühzeitig erkennen. Die für eine Störquelle typischen Veränderungen der Messsignale können somit bereits frühzeitig erkannt werden, ohne dass der Fadenspannungsmesswert zuvor einen Schwellwert überschreitet. Die Abhängigkeit der Messsignale voneinander aufgrund einer Störquelle treten unabhängig von einem Schwellwert der Fadenspannung ein. So können aus einer Analyse der Messsignaländerungen heraus typische Merkmale zur Identifizierung der Störquelle abgeleitet werden. Das maschinelle Lernprogramm ermöglicht eine schnelle und komplexe Analyse einer Vielzahl an Messsignalen, so dass deren Abhängigkeiten zueinander insbesondere in zeitlichen Abständen schnell analysiert und somit eine Störquelle sicher und schnell identifizierbar ist.

Da die Veränderung der Messsignale aufgrund einer Störquelle in Abhängigkeit von der Störquelle individuelle charakteristische zeitliche Veränderungen verursachen, ist eine Verfahrensvariante besonders vorteilhaft, um möglichst eindeutige Identifizierungen der Störquelle zu erhalten. So wird die Folge der Messsignale der Fadenspannung bevorzugt als ein Analysegraph erfasst und analysiert.

Um insbesondere die zulässigen Änderungen der Fadenspannung von einer unzulässigen Änderung der Fadenspannung, die einen vorgegebenen Schwellwert überschreitet, differenziert behandeln zu können, ist die Verfahrensvariante besonders vorteilhaft, bei welcher eine zeitliche Folge von Messsignalen der Fadenspannung bei einer Überschreitung eines Schwellwertes der Fadenspannung als ein Fehlergraph erfasst und analysiert wird. Der Schwellwert kann beispielweise durch einen oberen Grenzwert und einen unteren Grenzwert der Fadenspannung auch einen Toleranzbereich umfassen.

Aufgrund einer Vielzahl von Messsignalen, die kontinuierlich zur Analyse anstehen, hat sich die Verfahrensvariante besonders bewährt, bei welcher die Analyse der Messsignale der Fadenspannung durch zumindest einen Maschinenlernalgorithmus des maschinellen Lernprogramms ausgeführt wird. So lässt sich eine künstliche Intelligenz dazu nutzen, um selbst bei einer großen Datenmenge strukturierte Analysen durchzuführen und die Identifizierung der Störquelle in kürzesten Analysezeiten zu ermöglichen. Hierzu ist es jedoch erforderlich, dass der Maschinenlernalgorithmus zum Lernen zunächst auf ermittelte Basisdaten zurückgreift. Hierzu werden beispielsweise zu Beginn eines Prozesses analysierte Analysegraphen und analysierte Fehlergraphen dem Maschinenlernalgorithmus zum Lernen übergeben. Nach einer Lernphase ist es möglich, dass der Maschinenlernalgorithmus durch Analyse der Messsignale oder Analysegraphen oder Fehlergraphen selbsttätig eine eindeutige Identifizierung der jeweiligen Störquelle ausführt.

Um bei einer Vielzahl von Störquellen die Identifizierung zu erleichtern, ist desweiteren vorgesehen, dass den Analysegraphen und/oder den Fehlergraphen mehrere Störungsgraphen zugeordnet sind, wobei jede der Störquellen durch einen der Störungsgraphen bestimmt ist. Derartige Störungsgraphen werden zur Schulung des Maschinenlernprogramms genutzt, so dass bei der Analyse von Analysegraphen oder Fehlergraphen frühzeitige Identifizierungen der Störquelle möglich sind.

Gemäß einer besonders vorteilhaften Verfahrensvariante erreicht der Maschinenlernalgorithmus nach einer Lernphase einen Betriebsstatus einer abgeschlossenen Schulung. Die Zuführung von Störungsgraphen kann entfallen und das Maschinenlernprogramm ist in der Lage die gemessenen Fehlergraphen zu analysieren und ggf. einer bekannten Störquelle zuordnen.

Für den Fall, dass unbekannte Störquellen auftreten, die nicht identifizierbar sind, lässt sich der Maschinenlernalgorithmus zur externen Schulung auswechseln, um die Produktion mit neu geschultem Maschinenlernalgorithmus fortsetzen zu können. Insoweit lässt sich das Diagnosesystem ständig auf neue, noch nicht bekannte Störquellen erweitern.

Hierbei sind als Störquellen Bedienungsfehler oder Fehleinstellungen eines Prozessaggregates oder Materialfehler oder Verschleiß eines Fadenführungselementes oder ein Fadenknoten oder sonstige Produktfehler identifizierbar. Die frühzeitige Diagnose möglicher Störquellen bereits vor Erreichen eines Schwellwertes der Fadenspannung ist somit besonders geeignet, um beispielsweise eine vorausschauende Wartung an Prozessaggregaten auszuführen. So können beispielsweise Verschleißerscheinungen der Fadenführungselemente vorzeitig gegengewirkt werden.

Zur Automatisierung des jeweiligen Texturierprozesses ist es jedoch auch möglich, dass nach Identifizierung der Störquelle oder nach Zuordnung zu einem der Störungsgraphen ein Steuerungsbefehl zu einer Prozessänderung ausgelöst wird. Die Prozessänderung könnte beispielsweise ein vorzeitiger Spulenwechsel sein, um das Einwickeln eines Fadenknotens zu vermeiden. Alternativ besteht jedoch auch die Möglichkeit, über eine Signalgebung den Operator eine bestimmte Bedienungsanweisung zukommen zu lassen.

Die erfindungsgemäße Vorrichtung zur Überwachung des Texturierprozesses zur Herstellung gekräuselter Fäden löst die Aufgabe dadurch, dass die Datenanalyseeinrichtung durch eine Diagnoseeinheit gebildet ist, durch welche die Messsignale der Fadenspannung mit einem maschinellen Lernprogramm zum Identifizieren einer von mehreren Störquellen analysierbar sind. So können die im Zeitintervall auftretenden Messsignale der Fadenspannung in dem Texturierprozess unmittelbar zu einer Diagnose einer Störquelle genutzt werden.

Die Diagnoseeinheit weist zumindest einen programmierbaren Lernprozessor zur Ausführung des maschinellen Lernprogramms auf. Dabei kann der Lernprozessor direkt mit der Fadenspannungsmesseinrichtung gekoppelt werden.

Zur Optimierung des maschinellen Lernprogramms und zur Verbesserung der Diagnosesicherheit ist desweiteren vorgesehen, dass der Lernprozessor wahlweise zum Zwecke einer Schulung mit einer Eingabeeinheit gekoppelt ist, durch welche ein oder mehrere Störungsgraphen einlesbar sind. So werden insbesondere zum Lernen typische Störungsgraphen dem Maschinenlernprogramm zugeführt. Nach einer Lernphase und Abschluss einer Schulung ist der Lernprozessor ohne Verbindung zu der Eingabeeinheit einsatzbereit.

Damit bei der Prozessführung ein Operator über den jeweiligen Prozesslauf informiert ist, wird die vorteilhafte Weiterbildung der erfindungsgemäßen Vorrichtung genutzt, bei welcher der Lernprozessor mit einer Ausgabeeinheit gekoppelt ist, durch welche eine Identifizierung einer der Störquellen oder eine Zuordnung der analysierten Fehlergraphen zur einem Störungsgraphen visualisierbar ist. Diese Ausgabeeinheit kann dabei vorteilhaft drahtlos mit dem Lernprozessor gekoppelt sein und jede Art von Gerät darstellen, an welchem eine Anzeige möglich ist.

Um ein möglichst autarkes System zur Diagnose zu erhalten, ist desweiteren vorgesehen, dass der Lernprozessor ein neuronales Netz zur Ausführung des maschinellen Lernprogramms aufweist. So können die großen Datenmengen an Messsignalen der Fadenspannung durch eine künstliche Intelligenz analysiert werden.

Zur Überwachung mehrerer Bearbeitungsstellen in einer Texturiermaschine ist die erfindungsgemäße Vorrichtung in der Weiterbildung vorteilhaft nutzbar, bei welchem der Lernprozessor räumlich getrennt zu der Eingabeeinheit und der Ausgabeeinheit angeordnet ist. Hierbei besteht die Möglichkeit, dass der Lernprozessor mit mehreren Eingabeeinheiten und insbesondere mit mehreren Ausgabeeinheiten in Kontakt steht. Die Verbindung kann drahtlos ausgeführt sein, so dass der Lernprozessor beispielsweise auch in einem virtuellen Raum ausgebildet sein könnte.

Zur Automatisierung wird die erfindungsgemäße Vorrichtungsvariante vorteilhaft verwendet, bei welcher die Diagnoseeinheit mit einer Maschinensteuereinheit verbunden ist, durch welche ein Steuerbefehl zur Prozessänderung ausführbar ist. So könnte beispielsweise nach Identifizierung eines Fadenknotens eine Maßnahme eingeleitet werden, um den Fadenknoten am Anfang oder am Ende einer gewickelten Spule einzuwickeln.

Das erfindungsgemäße Verfahren zur Überwachung eines Texturierprozesses wird nachfolgend anhand einiger Ausführungsbeispiele der erfindungsgemäßen Vorrichtung unter Bezug auf die beigefügten Figuren näher erläutert.

Es stellen dar:
- Fig. 1: schematisch ein Ausführungsbeispiel eines Texturierprozesses zur Herstellung eines gekräuselten Fadens
- Fig. 2: schematisch ein Ausführungsbeispiel der erfindungsgemäßen Vorrichtung zur Überwachung des Texturierprozesses gemäß Fig. 1
- Fig. 3: schematisch ein Fadenspannungsdiagramm mit einer Folge von Messsignalen
- Fig. 4: schematisch ein weiteres Ausführungsbeispiel der erfindungsgemäßen Vorrichtung zur Überwachung des Texturierprozesses gemäß Fig. 1
- Fig. 5.1: schematisch ein Analysegraph mit einer Zeitfolge mehrerer Messsignale der Fadenspannung
- Fig. 5.2: schematisch ein Fehlergraph in einer Zeitfolge mehrerer Messsignale der Fadenspannung
- Fig. 6.1 bis Fig. 6.5: schematisch mehrerer Störungsgraphen mit jeweils einem Messsignalverlauf der Fadenspannung verschiedener Störquellen

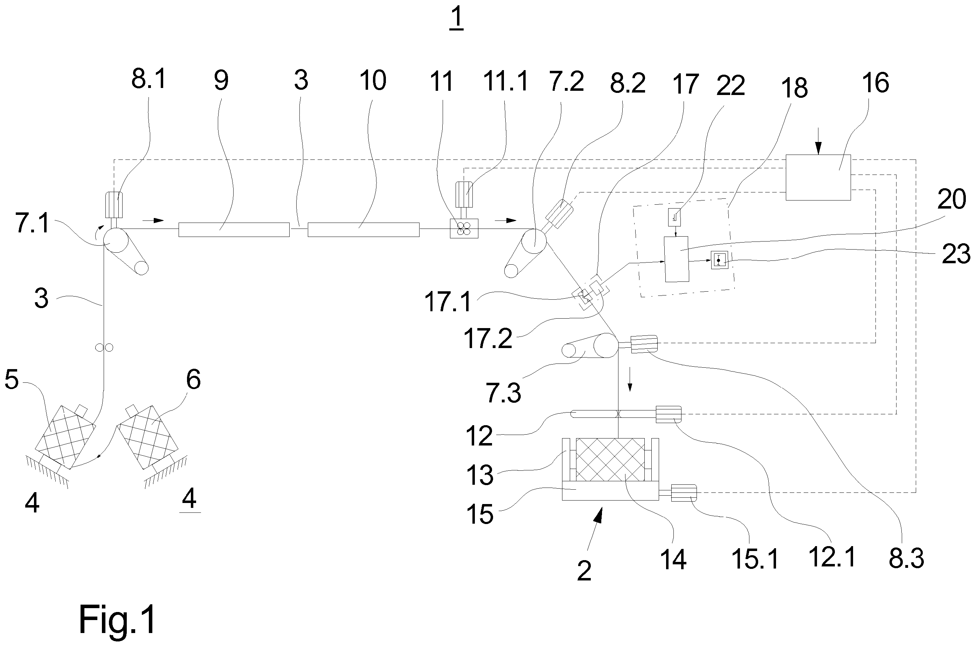

In der Fig. 1 ist schematisch ein Ausführungsbeispiel eines Texturierprozesses zur Herstellung eines gekräuselten Fadens gezeigt. Der Texturierprozess ist hierbei durch eine Bearbeitungsstelle einer Texturiermaschine beschrieben. Derartige Texturiermaschinen weisen üblicherweise eine Vielzahl von Bearbeitungsstellen auf, um eine Vielzahl von synthetischen Fäden parallel nebeneinander zu texturieren. In Fig. 1 ist schematisch eine Bearbeitungsstelle 1 und eine Spulstelle 2 einer derartigen Texturiermaschine dargestellt. Die Bearbeitungsstelle 1 weist ein Gatter 4 auf, in welcher eine Vorlagespule 5 und eine Reservespule 6 gehalten sind. Die Vorlagespule 5 liefert einen Faden 3, der zum Verstrecken und Texturieren in der Bearbeitungsstelle 1 überführt wird. Ein Fadenende der Vorlagespule 5 ist mit einem Fadenanfang der Reservespule 6 durch einen Fadenknoten miteinander verbunden. So wird ein kontinuierlicher Abzug des Fadens 3 nach Beendigung der Vorlagespule 5 realisiert. Das Fadenende der Reservespule 6 wird dann mit dem Fadenanfang einer neuen Vorlagespule 5 verbunden.

Der Abzug des Fadens 3 von der Vorlagespule 5 erfolgt durch ein erstes Lieferwerk 7.1. Das Lieferwerk 7.1 wird über einen Antrieb 8.1 angetrieben. Das Lieferwerk 7.1 ist in diesem Ausführungsbeispiel durch eine angetriebene Galette und eine frei drehbare Rolle gebildet, die mehrfach vom Faden umschlungen sind. Im weiteren Fadenverlauf ist dem Lieferwerk 7.1 eine Heizeinrichtung 9, eine Kühleinrichtung 10 und ein Texturieraggregat 11 nachgeordnet. Das Texturieraggregat 11 wird über einen Texturierantrieb 11.1 angetrieben. Das Texturieraggregat 11 ist vorzugsweise als ein Friktionsdrallgeber ausgeführt, um an dem Faden einen Falschdrall zu erzeugen, der sich in eine Kräuselung der einzelnen Filamente des Fadens auswirkt.

Zum Verstrecken des Fadens ist dem Texturieraggregat 11 ein zweites Lieferwerk 7.2 nachgeordnet, das durch den Antrieb 8.2 angetrieben wird. Das Lieferwerk 7.2 ist im Aufbau identisch zu dem ersten Lieferwerk 7.1, wobei das zweite Lieferwerk 7.2 mit einer höheren Umfangsgeschwindigkeit zum Verstrecken des Fadens betrieben wird. Innerhalb der Bearbeitungsstelle 1 wird der synthetische Faden 3 somit texturiert und gleichzeitig verstreckt. Nach dem Kräuseln des Fadens 3 wird dieser durch ein drittes Lieferwerk 7.3 zu einer Spulstelle 2 geführt. Das Lieferwerk 7.3 wird durch den Antrieb 8.3 angetrieben.

Die Spulstelle 2 weist einen Spulenhalter 13 auf, welcher eine Spule 14 trägt. Der Spulenhalter 13 ist schwenkbar ausgebildet und lässt sich zum Auswechseln der Spule 14 manuell oder automatisiert bedienen. Dem Spulenhalter 13 ist eine Treibwalze 15 zugeordnet, die durch einen Walzenantrieb 15.1 angetrieben wird. Zum Verlegen des Fadens am Umfang der Spule 15 ist der Spulstelle 2 eine Changiereinheit 12 zugeordnet, die einen antreibbaren Changierfadenführer aufweist. Der Changierfadenführer wird hierzu über den Changierantrieb 12.1 oszillierend angetrieben.

Der Changierantrieb 12.1 und der Walzenantrieb 15.1 der Spulstelle 2 sind als Einzelantriebe ausgebildet und mit einer Maschinensteuereinheit 16 verbunden. Ebenso sind die Antriebe 8.1, 8.2 und 8.3 der Lieferwerke 7.1, 7.2 und 7.3 sowie der Texturierantrieb 11.1 des Texturieraggregates 11 der Bearbeitungsstelle 1 als Einzelantriebe ausgeführt und mit der Maschinensteuereinheit 16 gekoppelt.

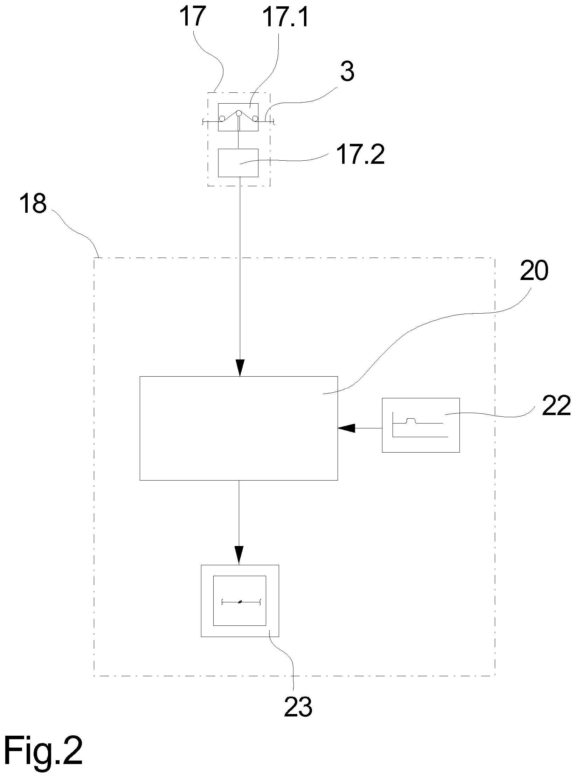

Zur Überwachung des Texturierprozesses wird an dem laufenden Faden 3 in einer Messstelle zwischen dem Lieferwerk 7.2 und 7.3 kontinuierlich eine Fadenspannung gemessen. Hierzu ist eine Fadenspannungsmesseinrichtung 17 vorgesehen, die einen Fadenspannungssensor 17.1 und einen Messsignalaufnehmer 17.2 aufweist. Die Fadenspannungsmesseinrichtung 17 ist mit einer als Diagnoseeinheit ausgebildete Datenanalyseeinrichtung 18 verbunden. Zur weiteren Erläuterung der Diagnoseeinheit 18 wird zusätzlich zu der Fig. 2 Bezug genommen.

In der Fig. 2 ist schematisch die Diagnoseeinheit 18 zur Analyse der Messsignale der Fadenspannung dargestellt. Die Diagnoseeinheit 18 umfasst in diesem Ausführungsbeispiel einen Lernprozessor 20. Der Lernprozessor 20 ist direkt mit dem Messsignalaufnehmer 17.2 der Fadenspannungsmesseinrichtung 17 verbunden. Der Lernprozessor 20 ist programmierbar ausgeführt und weist vorzugsweise ein neuronales Netz auf, um ein maschinelles Lernprogramm auszuführen. Das maschinelle Lernprogramm umfasst zumindest einen Maschinenlernalgorithmus, um mit künstlicher Intelligenz umfangreiche Analysen der Messsignale der Fadenspannung durchführen zu können.

Dem Lernprozessor 20 ist eine Eingabeeinheit 22 und eine Ausgabeeinheit 23 zugeordnet. Die Verbindung zwischen dem Lernprozessor 20 und der Fadenspannungsmesseinrichtung 17, der Eingabeeinheit 22 und der Ausgabeeinheit 23 kann durch jeweils eine drahtgebundene oder drahtlose Verbindung erfolgen. Insbesondere bei einer drahtlosen Verbindung besteht die Möglichkeit, dass einzelne Einheiten nicht unmittelbar an der Texturiermaschine gehalten werden müssen. So können auch Lernprogramme genutzt werden, die sich in einem virtuellen Raum befinden. So besteht die Möglichkeit, den Lernprozessor 20 unabhängig von der Eingabeeinheit 22 und der Ausgabeeinheit 23 anzuordnen.

In dem Lernprozessor 20 werden die von dem Messsignalaufnehmer 17.1 übermittelten Messsignale der Fadenspannung mit dem maschinellen Lernprogramm analysiert. Das maschinelle Lernprogramm weist zumindest einen Maschinenlernalgorithmus auf, der mit Hilfe eines neuronalen Netzes eine strukturierte Analyse der in einem Zeitintervall auftretenden Folge der Messsignale der Fadenspannung zur frühzeitigen Diagnose einer von mehreren Störquellen ausführt. Die in der zeitlichen Folge auftretenden Messsignaländerungen der Messsignale der Fadenspannung werden dabei analysiert, um typische Merkmale zur Identifizierung einer bestimmten Störquelle aufzudecken.

Bei dem in Fig. 2 dargestellten Ausführungsbeispiel ist dem Lernprozessor 20 die Eingabeeinheit 22 zugeordnet. Insoweit lässt sich der Maschinenlernalgorithmus kontinuierlich mit Störgraphen bekannter Störquellen schulen. Diese Lernphase des Maschinenlernalgorithmus erreicht in der Regel nach einer Betriebszeit einen Reifezustand und somit einen Betriebsstatus einer abgeschlossenen Schulung. In diesem Zustand wird die Diagnoseeinheit 18 ohne eine Eingabeeinheit 22 betrieben. Dementsprechend besteht auch die Möglichkeit, die Diagnoseeinheit 18 bei bekannten Texturierungsprozessen von Anfang an mit einem Lernprogramm mit geschultem Maschinenalgorithmus einzusetzen. Der Einsatz einer Eingabeeinheit wird dann entbehrlich.

Für den Fall, dass bei einem geschulten System ein Fehlergraph einer unbekannten Störquelle auftritt, der durch den Maschinenlernalgorithmus nicht identifizierbar ist, lässt sich das Lernprogramm des Lernprozessors Austauschen oder an einem zentralen Ort erneut schulen. Der Fehlergraph der unbekannten Störquelle wird zuvor direkt zum zentralen Schulungsort übermittelt, so dass vorhandene Maschinenlernalgorithmen bereits geschult werden können.

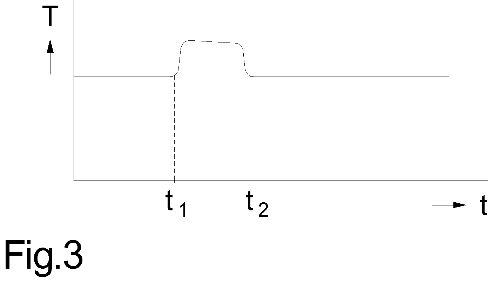

In der Fig. 3 ist ein Ausführungsbeispiel einer zeitlichen Folge von Messsignalen der Fadenspannung in einem Diagramm dargestellt. Die Höhe der Fadenspannung T ist an der Ordinate und die Zeit t an der Abszisse des Diagramms eingetragen. Der Verlauf der Messsignale lässt eine sprunghafte Fadenspannungserhöhung erkennen, die nur für einen kurzen Zeitintervall von der Zeit t₁ bis t₂ ansteht. Die Messsignale der Fadenspannung vor und nach dem Anstieg sind unauffällig. Diese Messsignalfolge deutet auf eine Störquelle in Form eines Fadenknotens hin, der die Messstelle durchlaufen hat. Zum Erkennen einer derartigen Störquelle wird der typische Messsignalverlauf zuvor dem Lernprogramm aufgegeben, so dass bei einer späteren Analyse von Messsignalfolgen derartige charakteristische Merkmale identifizierbar sind.

Für den Fall, dass bei der Überwachung des Texturierprozesses ein Fadenknoten im Faden die Messstelle passiert, wird durch den Messsignalaufnehmer 17.1 eine ähnliche Messsignalfolge erzeugt, die der Diagnoseinheit 18 zugeführt wird. Durch die im Lernprozessor durchgeführte Analyse der Messsignalfolge werden die typischen charakteristischen Messsignaländerungen identifiziert und die betreffende Störquelle erkannt. Hierbei ist es unerheblich, ob die Fadenspannungsänderung einen Schwellwert übersteigt oder innerhalb eines zulässigen Toleranzbereiches verbleibt.

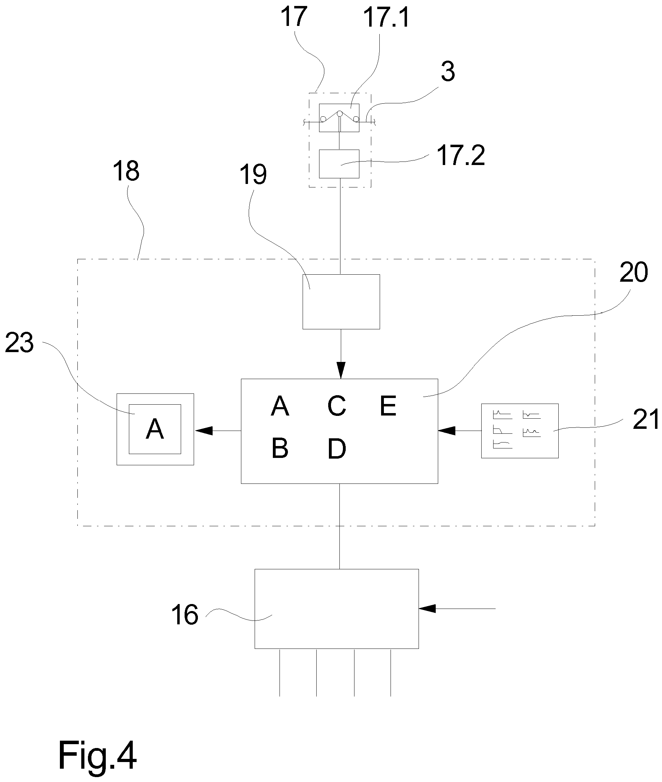

Da in einem Texturierprozess nicht nur Produktfehler als Störquelle auftreten, ist eine differenzierte und insbesondere eine erweiterte Analyse und Diagnose von Störquelle gewünscht. Hierzu ist in Fig. 4 ein weiteres Ausführungsbeispiel der erfindungsgemäßen Vorrichtung dargestellt, wie sie beispielsweise in dem Texturierprozess nach Fig. 1 einsetzbar wäre. In diesem Fall umfasst die Diagnoseeinheit 18 eine Fadenspannungsauswertungseinheit 19, die direkt mit der Fadenspannungsmesseinrichtung 17 verbunden ist. So werden die Messsignale des Messsignalaufnehmers 17.2 der Fadenspannungsauswertungseinheit 19 zugeführt. Innerhalb der Fadenspannungsauswertungseinheit 19 werden zunächst zeitliche Verläufe der Messsignale in einer Folge aufgezeichnet und als ein Analysegraph erzeugt. Parallel werden die Messsignale mit einem Schwellwert verglichen. Üblicherweise wird das Messsignal der Fadenspannung mit einem oberen Grenzwert und einem unteren Grenzwert der Fadenspannung verglichen. Sobald eine unzulässige Toleranzabweichung der Fadenspannung erkannt wird, wird der kurzzeitige Messsignalverlauf der Fadenspannung aufgezeichnet und als ein Fehlergraph erzeugt.

Je nachdem, ob ein Analysegraph ohne Schwellwertüberschreitung oder ein Fehlergraph mit Schwellwertverletzung vorliegt, wird dieser an einen Lernprozessor 20 übergeben. Der Lernprozessor 20 ist dementsprechend in seinem Maschinenlernalgorithmus angepasst, um entsprechende Analysen zur Diagnose der Störursache ausführen zu können.

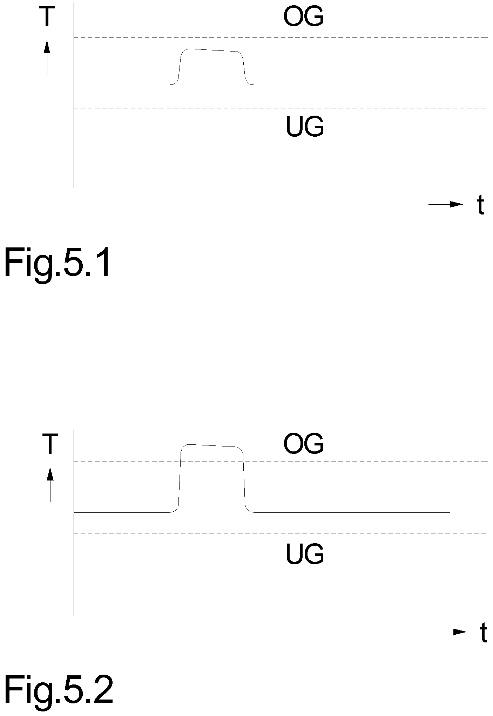

In den Fig. 5.1 und 5.2 ist jeweils ein Analysegraph ohne Schwellwertverletzung und ein Fehlergraph mit Schwellwertverletzung dargestellt. Das Messsignal der Fadenspannung wird dabei mit einem oberen Grenzwert und einem unteren Grenzwert verglichen. In dem Analysegraph und dem Fehlergraph ist der obere Grenzwert mit dem Kennbuchstaben OG und der unter Grenzwert mit dem Kennbuchstaben UG gekennzeichnet. Hierzu ist auf der Ordinate die Fadenspannung T und auf der Abszisse die Zeit t aufgetragen. Bei dem in Fig. 5.1 dargestellten Signalverlauf der Messsignale der Fadenspannung ist keine Überschreitung des oberen Grenzwertes und keine Unterschreitung des unteren Grenzwertes zu erkennen. Insoweit ist die durch eine Störquelle verursachte Signaländerung der Fadenspannung im zulässigen Bereich. Dennoch könnte beispielsweise ein Fadenknoten im Folgeprozess in einem Gewebe einen unzulässigen Fehler darstellen.

Bei dem in Fig. 5.2 dargestellten Fehlergraph übersteigen die Messsignale der Fadenspannung kurzzeitig den oberen Grenzwert OG, so dass eine Schwellwertverletzung und somit eine unzulässige Qualitätsverschlechterung des Fadens vorliegt. Das erfindungsgemäße Verfahren und die erfindungsgemäße Vorrichtung sind somit unabhängig davon, ob die durch eine Störquelle verursachte Prozessstörung eine zulässige oder unzulässige Fadenspannungsänderung an dem Faden verursacht.

Wie aus der Darstellung in Fig. 4 hervorgeht, werden dem Lernprozessor über die Eingabeeinheit eine Mehrzahl von sogenannten Störungsgraphen vorgegeben. Ein Störungsgraph beinhaltet dabei den für die bestimmte Störquelle charakteristischen Messsignalverlauf der Fadenspannung. So lässt sich das maschinelle Lernprogramm schulen und erweitern, um eine eindeutige Diagnose und Identifizierung der Störquellen zu ermöglichen

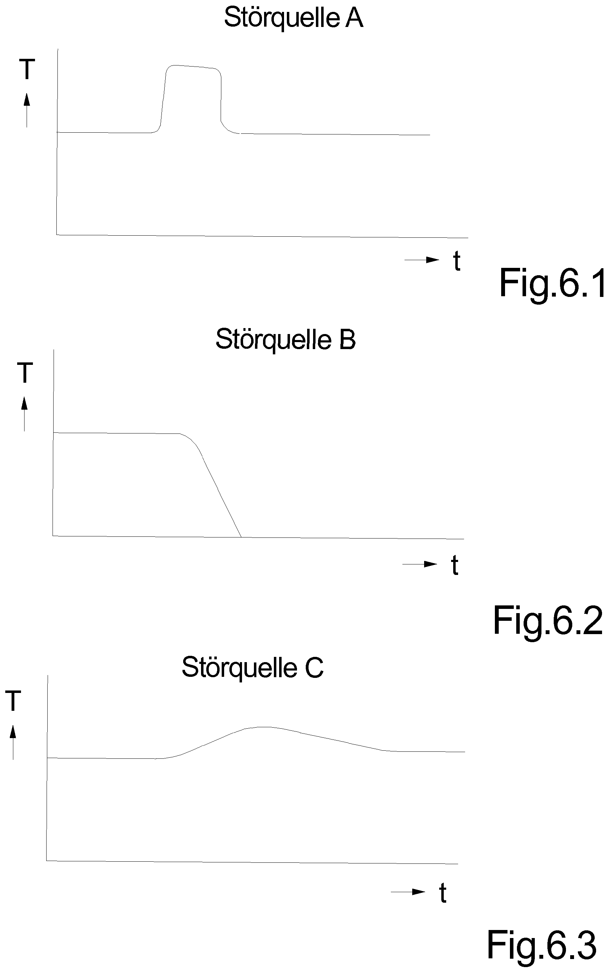

In den Fig. 6.1 bis 6.5 sind beispielhaft mehrere Störungsgraphen verschiedener Störquellen schematisch durch eine typische Messsignalfolge der Fadenspannung gekennzeichnet. Jeder der Störungsgraphen stellt eine typische Störquelle, wie sie in dem Texturierprozess auftreten können, dar. Der Störungsgraph nach Fig. 6.1 zeigt ein Signalverlauf der Fadenspannung bei einem Fadenknoten, die hier als Störquelle A gekennzeichnet ist.

Die in Fig. 6.2 dargestellte Störquelle B zeigt einen plötzlichen Fadenspannungsabfall, wie er beispielsweise durch einen Fadenbruch oder einen Bedienfehler verursacht sein könnte. Hierbei bricht die Fadenspannung an dem Fadenspannungssensor plötzlich komplett ein.

Der in Fig. 6.3 dargestellte Störungsgraph bestimmt die Störquelle C. Die Störquelle C könnte beispielsweise eine Verschleißsituation an einem der Prozessaggregate oder einem Fadenführungselement darstellen.

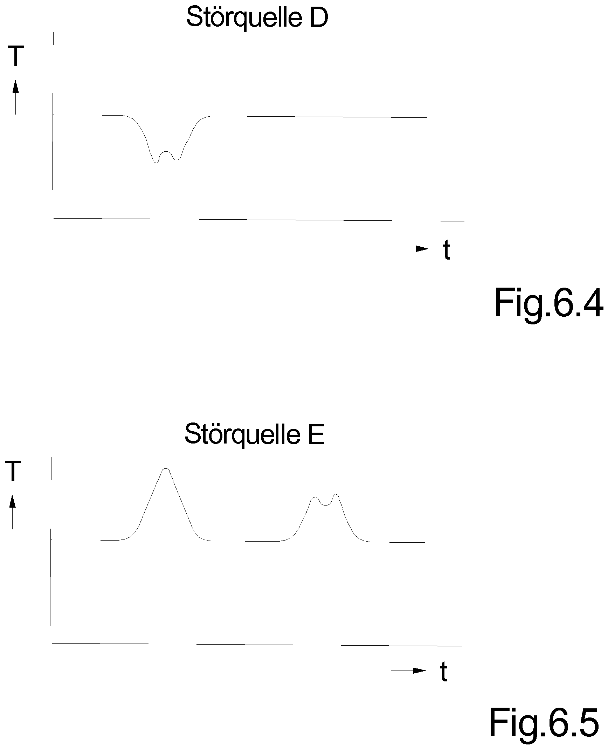

Bei dem in Fig. 6.4 dargestellten Störungsgraphen tritt ein kurzzeitiger Fadenspannungsverlust auf, der hier der Störquelle D zugeordnet ist. Hier könnte eine Prozessstörung beim Spulwechsel in der Spulstelle vorliegen.

In dem in Fig. 6.5 dargestellten Störungsgraphen ist eine wiederkehrende Überhöhung des Messsignales der Fadenspannung erkennbar. Der zugehörige Störungsgraph ist in diesem Fall der Störquelle E zugeordnet. Die Störquelle E könnte beispielsweise in einer ungleichmäßigen Texturierung des Fadens liegen.

Insoweit ist jedem der Störungsquellen A bis E eine bestimmte Prozessstörung oder ein bestimmter Bedienungsfehler oder ein bestimmter Produktfehler zugeordnet. Die in den Fig. 6.1 bis 6.5 dargestellten Störungsgraphen und deren Störquellen sind beispielhaft. In einem Texturierprozess, wie in Fig. 1 dargestellt, können eine Vielzahl von Störquellen auftreten, denen eine eindeutige Messsignalfolge zur Diagnoseidentifizierung zugrunde liegt.

Bei dem in Fig. 4 dargestellten Ausführungsbeispiel der erfindungsgemäßen Vorrichtung ist die Diagnoseinheit 18 über dem Lernprozessor 20 direkt mit einer Maschinensteuereinheit 16 verbunden. Die Maschinensteuereinheit 16 ist wie in Fig. 1 gezeigt, mit den Antrieben und Aktoren der Prozessaggregate der Bearbeitungsstelle 1 gekoppelt, die zur Durchführung des Texturierprozesses gesteuert und geregelt werden. Insoweit ist nach Identifizierung und Diagnose einer Störquelle unmittelbar ein Prozesseingriff möglich. So könnte beispielsweise bei Diagnose der Störquelle A, die einen Fadenknoten als Ursache hat, in der Spulstelle 2 ein Spulenwechsel initiiert werden, so dass der Fadenknoten am Ende oder am Anfang einer Spule gewickelt wird. Ebenso lassen sich durch frühzeitige Diagnosen einer Verschleißerscheinung entsprechende Wartungsarbeiten initiieren. So können vorausschauende Instandsetzungen und Wartungen an den Prozessaggregaten durchgeführt werden, um die Qualität bei der Herstellung des gekräuselten Fadens möglichst konstant zu halten. Das in Fig. 4 dargestellte Ausführungsbeispiel der erfindungsgemäßen Vorrichtung befindet sich noch in einer Lernphase. So bald die Schulung des Maschinenlernalgorithmus zur Identifizierung aller bekannter Störquellen abgeschlossen ist, lässt sich die Diagnoseeinheit 18 ohne eine Eingabeeinheit 22 betreiben. Dann werden die Fadenspannungssignale unmittelbar dem Lernprozessor 20 zugeführt und direkt analysiert.

## Patentansprüche

1. Verfahren zur Überwachung eines Texturierprozesses zur Herstellung gekräuselter Fäden, bei welchem an dem texturierten Faden fortlaufend eine Fadenspannung gemessen wird und bei welchem die Messsignale der Fadenspannung zumindest in einem Zeitintervall kontinuierlich erfasst und analysiert werden, **dadurch gekennzeichnet, dass** eine im Zeitintervall auftretende Folge der Messsignale der Fadenspannung zur frühzeitigen Diagnose einer von mehreren Störquellen mit einem maschinellen Lernprogramm analysiert wird.

2. Verfahren nach Anspruch 1, **dadurch gekennzeichnet, dass** die Folge der Messsignale der Fadenspannung als ein Analysegraph erfasst und analysiert wird.

3. Verfahren nach Anspruch 1, **dadurch gekennzeichnet, dass** die Folge von Messsignalen der Fadenspannung bei einer Überschreitung eines Schwellwertes der Fadenspannung als ein Fehlergraph erfasst und analysiert wird.

4. Verfahren nach einem der Ansprüche 1 bis 3, **dadurch gekennzeichnet, dass** die Analyse der Messsignale der Fadenspannung durch zumindest einen Maschinenlernalgorithmus des maschinellen Lernprogramms ausgeführt wird.

5. Verfahren nach Anspruch 4, **dadurch gekennzeichnet, dass** eine der Störquellen durch den Maschinenlernalgorithmus aus analysierten Folgen von Messsignalen oder aus analysierten Analysegraphen oder aus analysierten Fehlergraphen identifiziert wird.

6. Verfahren nach Anspruch 5, **dadurch gekennzeichnet, dass** den Analysegraphen und/oder den Fehlergraphen mehrere Störungsgraphen zugeordnet sind, wobei jede der Störquellen durch einen der Störungsgraphen bestimmt ist.

7. Verfahren nach einem der vorgenannten Ansprüche, **dadurch gekennzeichnet, dass** der Maschinenlernalgorithmus nach einer Lernphase einen Betriebsstatus einer abgeschlossenen Schulung erreicht hat.

8. Verfahren nach Anspruch 7, **dadurch gekennzeichnet, dass** der Maschinenlernalgorithmus bei einem Auftreten unbekannter Störquellen zum Zwecke einer externen Schulung ausgewechselt wird.

9. Verfahren nach einem der Ansprüche 1 bis 8. **dadurch gekennzeichnet, dass** ein Bedienungsfehler, eine Fehleinstellung eines Prozessaggregates, ein Materialfehler, ein Verschleiß eines Fadenführungselementes und/oder ein Fadenknoten eine der Störquellen sind.

10. Verfahren nach einem der Ansprüche 1 bis 9, **dadurch gekennzeichnet, dass** nach Identifizierung der Störquelle oder nach Zuordnung zu einen der Störungsgraphen ein Steuerungsbefehl zu einer Prozessänderung ausgelöst wird.

11. Vorrichtung zur Überwachung eines Texturierprozesses zur Herstellung gekräuselter Fäden mit einer Fadenspannungsmesseinrichtung (17) zum fortlaufenden Messen einer Fadenspannung an einer Messstelle und mit einer Datenanalyseeinrichtung (18) zur Analyse von zumindest in einem Zeitintervall kontinuierlich erfassten Messsignalen der Fadenspannungsmesseinrichtung (17), **dadurch gekennzeichnet, dass** die Datenanalyseeinrichtung durch eine Diagnoseeinheit (18) gebildet ist, die eingerichtet ist, eine im Zeitintervall auftretende Folge der Messsignale der Fadenspannung (T) mit einem maschinellen Lernprogramm zum Identifizieren einer von mehreren Störquellen zu analysieren.

12. Vorrichtung nach Anspruch 11, **dadurch gekennzeichnet, dass** die Diagnoseeinheit (18) einen programmierbaren Lernprozessor (20) zur Ausführung des maschinellen Lernprogramms aufweist.

13. Vorrichtung nach Anspruch 12, **dadurch gekennzeichnet,**
- **dass** der Lernprozessor (20) wahlweise zum Zwecke einer Schulung mit einer Eingabeeinheit (22) der Vorrichtung gekoppelt ist, durch welche ein oder mehrere ermittelte Störungsgraphen der Fadenspannung einlesbar sind, und/oder
- der Lernprozessor (20) mit einer Ausgabeeinheit (23) der Vorrichtung gekoppelt ist, durch welche eine Identifizierung der Störquelle und/oder eine Zuordnung zu einem der Störungsgraphen visualisierbar ist, und/oder
- die Diagnoseeinheit (18) mit einer Maschinensteuereinheit (16) der Vorrichtung verbunden ist, durch welche ein Steuerbefehl zur Prozessänderung ausführbar ist.

14. Vorrichtung nach einem der Ansprüche 12 oder 13, **dadurch gekennzeichnet, dass** der Lernprozessor (20) ein neuronales Netz zur Ausführung des maschinellen Lernprogramms aufweist.

15. Vorrichtung nach Anspruch 13, **dadurch gekennzeichnet, dass** der Lernprozessor (20) von der Eingabeeinheit (22) und der Ausgabeeinheit (23) räumlich getrennt ist.

## Claims

1. Method for monitoring a texturing process for producing crimped threads, in which a thread tension is measured continuously on the textured thread and in which the thread tension measuring signals are captured and analysed continuously, at least in one time interval, **characterized in that** a sequence of the thread tension measuring signals occurring in the time interval is analysed with a machine learning program for the early diagnosis of one of a plurality of fault sources.

2. Method according to Claim 1, **characterized in that** the sequence of the thread tension measuring signals is captured and analysed as an analysis graph.

3. Method according to Claim 1, **characterized in that** the sequence of thread tension measuring signals is captured and analysed as an error graph if a thread tension threshold value is exceeded.

4. Method according to one of Claims 1 to 3, **characterized in that** the analysis of the thread tension measuring signals is performed by at least one machine learning algorithm of the machine learning program.

5. Method according to Claim 4, **characterized in that** one of the fault sources is identified by the machine learning algorithm from analysed sequences of measuring signals or from analysed analysis graphs or from analysed error graphs.

6. Method according to Claim 5, **characterized in that** a plurality of fault graphs are assigned to the analysis graphs and/or the error graphs, wherein each of the fault sources is defined by one of the fault graphs.

7. Method according to one of the aforementioned claims, **characterized in that** the machine learning algorithm has attained an operational status of completed training after a learning phase.

8. Method according to Claim 7, **characterized in that** the machine learning algorithm is exchanged for the purpose of external training if unknown fault sources occur.

9. Method according to one of Claims 1 to 8, **characterized in that** an operating error, an incorrect setting of a process unit, a material defect, wear of a thread-guiding element and/or a thread knot are/is one of the fault sources.

10. Method according to one of Claims 1 to 9, **characterized in that** a control command for a process change is triggered following identification of the fault source or following assignment to one of the fault graphs.

11. Device for monitoring a texturing process for producing crimped threads with a thread tension measuring device (17) for continuously measuring a thread tension at a measuring station and with a data analysis device (18) for analysing measuring signals of the thread tension measuring device (17) captured continuously, at least in one time interval **characterized in that** the data analysis device is formed by a diagnostic unit (18) configured to analyse a sequence of the thread tension (T) measuring signals occurring in the time interval with a machine learning program for identifying one of a plurality of fault sources.

12. Device according to Claim 11, **characterized in that** the diagnostic unit (18) has a programmable learning processor (20) to run the machine learning program.

13. Device according to Claim 12, **characterized**
- **in that** the learning processor (20) is optionally coupled for training purposes to an input unit (22) of the device by means of which one or more determined thread tension fault graphs are loadable, and/or
- the learning processor (20) is coupled to an output unit (23) of the device by means of which an identification of the fault source and/or an assignment to one of the fault graphs are/is visualizable, and/or
- the diagnostic unit (18) is connected to a machine control unit (16) of the device by means of which a control command for the process change is executable.

14. Device according to either of Claims 12 or 13, **characterized in that** the learning processor (20) has a neural network to run the machine learning program.

15. Device according to Claim 13, **characterized in that** the learning processor (20) is physically separated from the input unit (22) and the output unit (23).

## Revendications

1. Procédé de surveillance d'un processus de texturation pour la fabrication de fils frisés, lors duquel une tension de fil est mesurée en continu au niveau des fils texturés et lors duquel les signaux de mesure de la tension de fil sont continuellement acquis et analysés au moins dans un intervalle de temps, **caractérisé en ce qu'**une séquence des signaux de mesure de la tension de fil qui se produit dans l'intervalle de temps et analysée avec un programme d'apprentissage automatique en vue d'un diagnostic précoce d'une parmi plusieurs sources de perturbation.

2. Procédé selon la revendication 1, **caractérisé en ce que** la séquence des signaux de mesure de la tension de fil est acquise et analysée sous la forme d'un graphe d'analyse.

3. Procédé selon la revendication 1, **caractérisé en ce que** la séquence des signaux de mesure de la tension de fil, dans le cas d'un dépassement d'une valeur de seuil de la tension de fil, est acquise et analysée sous la forme d'un graphe des défauts.

4. Procédé selon l'une des revendications 1 à 3, **caractérisé en ce que** l'analyse des signaux de mesure de la tension de fil est réalisée par au moins un algorithme d'apprentissage automatique du programme d'apprentissage automatique.

5. Procédé selon la revendication 4, **caractérisé en ce que** l'une des sources de perturbation est identifiée par l'algorithme d'apprentissage automatique à partir des séquences analysées de signaux de mesure ou à partir des graphes d'analyse analysés ou à partir des graphes de défauts analysés.

6. Procédé selon la revendication 5, **caractérisé en ce que** plusieurs graphes de perturbations sont associés aux graphes d'analyse et/ou aux graphes de défauts, chacune des sources de perturbation étant déterminée par l'un des graphes de perturbations.

7. Procédé selon l'une des revendications précédentes, **caractérisé en ce que** l'algorithme d'apprentissage automatique a atteint une formation terminée après une phase d'apprentissage d'un état opérationnel.

8. Procédé selon la revendication 7, **caractérisé en ce que** l'algorithme d'apprentissage automatique, dans le cas d'une survenance de sources de perturbations inconnues, est remplacé en vue d'une formation externe.

9. Procédé selon l'une des revendications 1 à 8, **caractérisé en ce qu'**une erreur de commande, un réglage incorrect d'un groupe de processus, un défaut matériel, une usure d'un élément de guidage de fil et/ou un noeud dans le fil sont l'une des sources de perturbation.

10. Procédé selon l'une des revendications 1 à 9, **caractérisé en ce qu'**après l'identification de la source de perturbation ou après l'association à l'un des graphes de perturbations, une instruction de commande est déclenchée en vue d'une modification du processus.

11. Arrangement de surveillance d'un processus de texturation pour la fabrication de fils frisés, comprenant un dispositif de mesure de tension de fil (17) destiné à mesurer en continu une tension de fil à un point de mesure et comprenant un dispositif d'analyse de données (18) destiné à analyser les signaux de mesure du dispositif de mesure de tension de fil (17), acquis continuellement au moins dans un intervalle de temps, **caractérisé en ce que** le dispositif d'analyse de données est formé par une unité de diagnostic (18), qui est conçue pour analyser une séquence des signaux de mesure de la tension de fil (T) qui se produit dans l'intervalle de temps avec un programme d'apprentissage automatique en vue d'identifier une parmi plusieurs sources de perturbation.

12. Arrangement selon la revendication 11, **caractérisé en ce que** l'unité de diagnostic (18) possède un processeur d'apprentissage (20) programmable destiné à exécuter le programme d'apprentissage automatique.

13. Arrangement selon la revendication 12, **caractérisé en ce que**
- le processeur d'apprentissage (20) est connecté sélectivement, à des fins d'une formation, à une unité d'entrée (22) de l'arrangement, par laquelle peuvent être chargés un ou plusieurs graphes de perturbations déterminés de la tension de fil, et/ou
- le processeur d'apprentissage (20) est connecté à une unité de sortie (23) de l'arrangement, par laquelle peut être visualisée une identification de la source de perturbation et/ou une association à l'un des graphes de perturbations, et/ou
- l'unité de diagnostic (18) est reliée à une unité de commande de machine (16) de l'arrangement, par laquelle peut être exécutée une instruction de commande en vue d'une modification du processus.

14. Arrangement selon l'une des revendications 12 ou 13, **caractérisé en ce que** le processeur d'apprentissage (20) possède un réseau neuronal destiné à exécuter le programme d'apprentissage automatique.

15. Arrangement selon la revendication 13, **caractérisé en ce que** le processeur d'apprentissage (20) est séparé dans l'espace de l'unité d'entrée (22) et de l'unité de sortie (23).
